# FASCICULE DE BREVET EUROPEEN

(11) **EP 0 837 699 B1**
(45) Date de publication et mention de la délivrance du brevet: **21.05.2003**
(21) Numéro de dépôt: 96924965.5
(22) Date de dépôt: 10.07.1996
(51) Int. Cl.: A61K 47/48

(54) **IMMUNOVECTEURS UTILISABLES POUR LE TRANSPORT INTRACELLULAIRE ET INTRANUCLEAIRE**
IMMUNOVEKTOREN ZUM TRANSPORT IN DIE ZELLE UND DEN ZELLKERN
IMMUNOVECTORS FOR THE INTRACELLULAR AND INTRANUCLEAR TRANSPORT

(30) Priorité: 10.07.1995 FR 9508316
(43) Date de publication de la demande: 29.04.1998
(73) Titulaire: INSTITUT PASTEUR, 75724 Paris Cédex 15 (FR); UNIVERSITE PIERRE ET MARIE CURIE PARIS VI, 75252 Paris Cédex 05 (FR)
(72) Inventeur: AVRAMEAS, Stratis, F-75015 Paris (FR); BUTTIN, Gérard, F-75013 Paris (FR); TERNYNCK, Thérèse, F-75015 Paris (FR); NATO, Faridabano, F-92160 Antony (FR)
(74) Mandataire: Desaix, Anne
(86) Numéro de dépôt international: FR9601076
(87) Numéro de publication internationale: WO97002840

(56) Documents cités:
- EP-A- 0 468 637
- EP-A- 0 511 011
- WO-A-92/13570
- WO-A-94/04690
- US-A- 4 650 675
- NUCLEIC ACIDS RES. (1994), 22(25), 5530-9 CODEN: NARHAD;ISSN: 0305-1048, XP002021247 NIEMEYER, CHRISTOF M. ET AL: "Oligonucleotide-directed self-assembly of proteins: semisynthetic DNA-streptavidin hybrid molecules as connectors for the generation of macroscopic arrays and the construction of supramolecular bioconjugates"
- DATABASE CHEMABS CHEMICAL ABSTRACTS SERVICE, COLUMBUS, OHIO, US AN122:78416, KHAN, SHABBIR A.: "Regulation of human immunodeficiency virus gene expression by the Tat and Rev proteins" XP002021248 & PEPTIDES (1994), 279-300. EDITOR(S): BASAVA, CHANNA;ANANTHARAMAIAH, GATTADAHALLI NANJUNATH. PUBLISHER: BIRKHAEUSER, BOSTON, MASS. CODEN: 60JMAN,

## Description

La présente invention est relative au transfert actif d'haptènes, de protéines, d'acides nucléiques et autres molécules dans le noyau de cellules eucaryotes. Cette invention revêt une importance majeure puisqu'elle peut être appliquée à divers domaines, notamment celui de la thérapie génique et des vaccins.

La thérapie génique demeure conditionnée par un nombre considérable de paramètres, parmi lesquels le développement de vecteurs capables de transporter à travers le cytoplasme de ces cellules de l'organisme de l'hôte des principes actifs doués de propriétés spécifiques prédéterminées dans les noyaux de cellules de l'organisme en l'absence d'altérations génétiques associées à l'utilisation de ces vecteurs, et la non-dégradation de l'activité biologique des principes actifs transférés. On sait qu'à ce jour toutes ces conditions sont loin d'être réunies (1).

En effet, les méthodes actuelles utilisées couramment pour transférer l'ADN dans les cellules sont les suivantes : des méthodes générales, non sélectives, qui utilisent la propriété de l'ADN de coprécipiter avec le phosphate de calcium ou le DEAE-dextran, ou bien l'introduction directe de l'ADN dans les cellules sous l'effet d'un champ électrique (électroporation). Ces méthodes sont très toxiques pour les cellules, entraînant une grande mortalité et une grande variabilité selon les cellules utilisées. D'autres méthodes utilisent un ciblage de l'entrée du gène dans les cellules par des récepteurs présents à leur membrane L'ADN peut alors pénétrer dans la cellule par l'intermédiaire soit d'un ligand spécifique de ces récepteurs asialorosomucoide (2), insuline (3) ou transferrine (4), soit d'anticorps spécifiques de constituants membranaires (5). Le complexe ADN/ligand pénètre dans la cellule par un processus d'endocytose. La transfection est donc limitée par une destruction importante du complexe dans les vésicules lysosomales, et différentes méthodes ont été proposées pour pallier ces inconvénients, notamment le blocage du compartiment lysosomal par la chloroquine ou l'addition simultanée d'adénovirus qui échappent au compartiment endosomal en détruisant la membrane des vésicules d'endocytose (6).

La présente invention a pour but de mettre à disposition un nouveau type de vecteurs à la fois plus efficaces et d'une plus grande innocuité que les vecteurs viraux dont l'utilisation est envisagée à ce jour

L'invention concerne donc un produit de couplage entre un principe biologiquement actif et un de ces nouveaux vecteurs, ci-après dénommés "immunovecteurs", ledit produit de couplage étant caractérisé, à la fois par la capacité de l'immunovecteur à permettre l'internalisation dans des cellules eucaryotes de principes biologiquement actifs liés de manière covalente ou non covalente à ces immunovecteurs, et par leur affinité pour l'ADN de ces cellules, au point de rendre ledit immunovecteur apte à transférer le principe biologiquement actif à proximité immédiate des noyaux de ces cellules ou dans les noyaux de ces cellules

Ces immunovecteurs consistent de préférence en des anticorps ou fragments d'anticorps aptes à reconnaître des séquences d'ADN au sein de ces cellules, et auxquels peuvent être liés de manière covalente ou non covalente des principes biologiquement actifs, ces anticorps ou fragments d'anticorps étant en outre capables de transporter in vitro et in vivo ces principes biologiquement actifs à travers les membranes et le cytoplasme de ces cellules, et à les transférer à proximité ou même dans le noyau de ces cellules.

Il est entendu que dans la présente description le terme "principe biologiquement actif" concerne toute molécule, macromolécule, groupe de molécules possédant l'activité biologique du genre en question.

L'invention est également relative à un procédé de transfert, notamment d'haptènes, de protéines et/ou d'acides nucléiques dans le noyau de cellules particulièrement de cellules eucaryotes, ce procédé étant basé sur l'utilisation des propriétés desdits immunovecteurs.

L'existence d'anticorps capables de pénétrer à l'intérieur de noyaux de lymphocytes humains lorsque ces cellules sont incubées in vitro dans un milieu de culture contenant un sérum provenant de malades atteints de lupus érythémateux disséminé (LED), a été rapportée pour la première fois par Alarcon-Segovia et al. en 1978 (7). Par la suite, la même équipe a démontré que ces anticorps sont d'isotype IgG et sont capables de réagir avec des acides ribonucléiques, libres ou complexés avec des protéines (8). Récemment, ce type d'anticorps a été détecté chez la souris lupique MRL lpr/lpr, mais également chez la souris NZB présentant un syndrome de maladie hémolytique auto-immune et même chez la souris normale BALB/c. Certains anticorps monoclonaux, préparés à partir de la rate de ces souris, se sont avérés capables de pénétrer *in vitro* dans le noyau de cellules maintenues en culture (10-13). Comme chez l'humain, il a été noté que ces anticorps monoclonaux étaient capables de reconnaître des acides nucléiques. De plus, il a été montré que ces anticorps sont aussi capables, lorsqu'ils sont injectés chez la souris, de pénétrer dans plusieurs types de cellules, pour se retrouver dans leurs noyaux (11).

L'invention découle de la découverte que ce type d'anticorps ou des fragments de ces anticorps pouvaient aussi être utilisés comme vecteurs, ci-après « immunovecteurs » aptes à transporter des principes biologiquement actifs, tels que des haptènes, des protéines, et des acides nucléiques à travers les membranes et le cytoplasme des cellules correspondantes, et à assurer leur transfert dans le noyau desdites cellules.

Ces anticorps peuvent être obtenus sous forme polyclonale à partir d'un sérum, en particulier d'un animal préalablement immunisé contre des fragments d'acide nucléique porteurs de l'épitope correspondant, ou sous forme monoclonale, à partir d'hybridomes sécréteurs de tels anticorps.

Tout type de liaison, chimique ou non, peut être mise en oeuvre pour assurer le couplage d'un immunovecteur du type anticorps ou fragment d'anticorps ayant une affinité pour les acides nucléiques au principe biologiquement actif, par exemple un haptène ou un acide nucléique, aux fins de le transporter à travers les membranes et le cytoplasme des cellules, et à assurer le transfert de ces principes actifs dans le noyau.

De façon préférée l'on aura recours à un mode de couplage chimique, permettant la réalisation de liaisons covalentes ou non covalentes

Des produits de couplage préférés sont ceux dont les immunovecteurs sont sélectionnables par un test de pénétration cellulaire comportant une première incubation de l'immunovecteur étudié en présence de cellules en culture dans le noyau desquelles le principe actif susceptible d'être associé à l'immunovecteur doit être transporté, suivie, après fixation et perméabilisation de ces cellules, d'une autre incubation avec des anticorps anti-immunovecteurs marqués, et enfin d'une détection à proximité immédiate du noyau ou même au sein de celui-ci de la réaction immunologique du type antigène-anticorps entre l'immunovecteur et l'anticorps anti-immunovecteur.

Parmi les immunovecteurs préférés de la présente invention, on mentionnera les anticorps ayant une affinité pour un acide nucléique. ou un fragment de ceux-ci, ce dernier conservant cette affinité.

Les anticorps ayant également la capacité de se fixer sur les cellules, en particulier des cellules lymphoïdes sont également utilisables Cette dernière catégorie d'immunovecteurs peut également être sélectionnée par un test, lequel peut alors aussi comprendre l'incubation des immunovecteurs à l'étude avec des cellules lymphoïdes, le lavage desdites cellules lymphoïdes, l'incubation de ces dernières avec des anticorps anti-immunovecteurs marqués, et la détermination du nombre de cellules positives dans chaque population.

Dans une réalisation, les cellules lymphoïdes utilisées sont des splénocytes de souris auto-immunes présentant un syndrome lupique.

Un immunovecteur préféré pour le produit de couplage est choisi parmi les IgG monoclonales, les fragments (Fab')2 ou (Fab'), ou tous polypeptides correspondant au(x) site(s) des anticorps impliqué(s) dans la reconnaissance de l'acide nucléique correspondant

Préférentiellement, cet immunovecteur est une immunoglobuline, plus particulièrement une IgG portant une activité anti-ADN, et provenant d'individus normaux.

En outre, cet immunovecteur peut être une IgG portant une activité anti-ADN, et provenant d'individus présentant des syndromes auto-immuns, plus particulièrement des syndromes de lupus érythémateux disséminé

Dans une réalisation particulière de l'invention, l'immunovecteur couplé au principe actif est un anticorps bi-spécifique reconnaissant d'une part l'ADN, et d'autre part une protéine telle que Tat, Rev du rétrovirus VIH, ainsi que des marqueurs de surface tels que CD3, CD4, CD8, CD19, et CD34.

Préférentiellement, le principe biologiquement actif couplé à l'immunovecteur est une molécule choisie parmi notamment des acides nucléiques, des protéines notamment les enzymes, par exemple la peroxydase, des haptènes notamment la biotine ou la fluorescéine, les activateurs ou inhibiteurs d'enzymes et les médicaments.

Dans une réalisation préférée, l'acide nucléique couplé est un polynucléotide, l'immunovecteur étant une IgG, le couplage s'effectuant par l'intermédiaire de la p-benzoquinone à raison d'une molécule d'immunovecteur pour 4 molécules de polynucléotide.

Un principe biologiquement actif utilisé de préférence, est constitué par un plasmide destiné à s'intégrer dans le noyau des cellules cibles pour l'expression d'une protéine codée par un gène contenu dans ledit plasmide. Lorsqu'un tel plasmide est couplé à un immunovecteur du type anticorps, ayant de l'affinité pour l'ADN, cet immunovecteur est, de façon préférée, préalablement couplé à un agent capable d'induire un effet de compactage sur l'ADN, cet agent étant de préférence la polylysine.

En effet, la polylysine, qui par ses propriétés cationiques est capable de compacter l'ADN, favorise la transfection des cellules. Le couplage de polylysine à l'immunovecteur, qui s'effectue à l'aide d'un agent de couplage, plus particulièrement un carbodiimide tel que l'EDC (1-(3-dimethylaminopropyl)-1'-ethyl carbodiimide), permet à l'ADN de réagir avec celle-ci. Cela a pour effet d'induire la libération du site actif de l'anticorps qui peut être parfois masqué lorsque l'anticorps est couplé à un principe actif de la taille d'un plasmide.

D'autres agents susceptibles d'avoir, grâce à leurs propriétés cationiques. un effet de compactage sur l'ADN (2-6), peuvent aussi être couplés à l'immunovecteur pour remplir une telle fonction.

De manière plus spécifique, un principe biologiquement actif utilisé préférentiellement est constitué par un gène destiné à s'intégrer dans le génome des cellules cibles, notamment par recombinaison homologue. plus particulièrement un ADN "nu" contenant une séquence d'acide nucléique codant pour un polypeptide originaire de cellules bactériennes ou eucaryotes, de cellules fongiques, ou de virus, ce polypeptide ayant des propriétés vaccinantes.

Avantageusement, ce principe actif permet l'immortalisation de types cellulaires choisis, particulièrement les macrophages, les cellules dendritiques, les cellules B et T, et les cellules hématopoiétiques, notamment d'origine humaine

De façon encore préférée, ce principe biologiquement actif est un oligonucléotide antisens permettant l'inhibition de la synthèse protéique ou nucléotidique, par exemple dans les cellules infectables par un rétrovirus VIH, ou dans des cellules tumorales.

Ainsi, les inventeurs ont d'une part tenté d'obtenir à partir de la rate de souris autoimmunes (NZB x NZW)F1, présentant un syndrome lupique, des anticorps monoclonaux IgG qui ont été sélectionnés pour leur capacité à réagir avec l'ADN mais aussi, pour leur capacité à pénétrer jusqu'au noyau des cellules. Parallèlement, des anticorps polyclonaux réagissant avec l'ADN et pouvant pénétrer dans les noyaux des cellules ont été isolés par chromatographie d'affinité Cette chromatographie a été appliquée, soit à un "pool" de sérums de patients normaux ou à un sérum individuel normal, et de préférence à des sérums provenant de patients atteints d'infections, particulièrement à un sérum de patients normaux atteints de LED, soit à un sérum de souris (NZB x NZW)F1.

Dans un procédé de sélection d'immunovecteurs selon la présente invention, le test de pénétration cellulaire des immunovecteurs comprend une première incubation de lignées cellulaires eucaryotes dans un milieu contenant lesdits immunovecteurs de préférence en concentration croissante, puis la fixation et, si besoin, la perméabilisation, ou vice-versa, de ces cellules, suivie d'une incubation desdites lignées cellulaires avec des anticorps anti-immunovecteurs marqués de préférence à la fluorescéine ou à la peroxydase, et la localisation des anticorps ainsi marqués à proximité des noyaux desdites cellules ou mieux encore à l'intérieur des noyaux. Les lignées cellulaires sont notamment choisies parmi les fibroblastes, les thymocytes ou les splénocytes

Sans que les conditions réactionnelles qui suivent aient un caractère limitatif, il peut-être mentionné que la première incubation est souvent effectuée à 37°C, durant environ 2 à 8 heures avec des concentrations d'immunovecteurs d'environ 1 à 70 µg/ml, sur des lignées cellulaires ensemencées à une concentration allant de 5 x 10³ à 5 x 10⁶ cellules par millilitre

Dans une des réalisations du procédé, la lignée cellulaire est une lignée fibroblastique en croissance exponentielle ensemencée à une concentration de 2 x 10⁴ cellules par millilitre, ou des thymocytes ou des splénocytes de souris BALB/c lesquels sont mis en suspension à raison d'environ 10⁶ cellules par millilitre.

Par ailleurs, l'invention concerne un procédé de préparation de produit de couplage immunovecteur-molécule(s), les immunovecteurs étant choisis parmi les anticorps, plus particulièrement les IgG obtenus selon le procédé de sélection, les fragments (Fab')2 ou (Fab'), ou tout polypeptide correspondant au site des anticorps ou fragments d'anticorps impliqués dans le transport des molécules.
Dans le procédé de préparation d'un produit de couplage selon l'invention, il est fait en sorte qu'à chaque immunovecteur est couplée au moins une molécule de produit biologiquement actif, ladite molécule étant liée à l'immunovecteur de préférence de façon covalente.

Les exemples qui suivent illustrent des conditions dans lesquelles, des haptènes comme la fluorescéine et la biotine, des petites molécules comme des hormones, des protéines notamment des enzymes, des inhibiteurs ou activateurs d'enzymes et des médicaments, par exemple des antiviraux tels que l'acyclovir ou l'AZT, peuvent être activement transportées à travers le cytoplasme des cellules traitées et transférées dans le noyau desdites cellules. En particulier, la fluorescéine a été couplée aux groupements aminés libres des immunovecteurs par l'intermédiaire d'un groupement actif isothioyanate et la biotine par l'intermédiaire d'un ester succinimide actif. Le couplage des haptènes ou autres à l'immunovecteur peut être effectué par l'intermédiaire d'autres réactifs ou groupes de pontage homo- ou héterobifonctionnels connus dans la littérature comme les imidoesters et N-hydroxy succinimydil esters pouvant réagir avec les groupements aminés, par exemple des dérivés de groupes alkyle, haloaryle, haloacetyl et pyridyl disulfure réagissant préférentiellement maléimides, ou par l'intermédiaire de avec des groupements sulfhydryle, des carbodiimides, ainsi que des molécules portant des groupements photoactivables comme l'azidobenzoyl hydrazide (13).

En outre, les inventeurs ont préparé des produits de couplage où l'immunovecteur est un anticorps bi-spécifique contre un antigène cible, construit, soit par voie chimique soit par voie du génie génétique, et ont parallèlement immunisé des individus, par exemple des souris avec lesdits antigènes cibles, et sélectionné des immunovecteurs anticorps bi-spécifiques réagissant préférentiellement avec lesdits antigènes cibles, de sorte que l'action des immunovecteurs soit spécifiquement dirigée.

Des techniques relatives à la synthèse d'anticorps bi-spécifiques ont été notamment décrites par Porstmann et al en 1984 (16), une étude intitulée "Development of a bispecific monoclonal antibody for use in molecular hybridisation" ayant été par ailleurs publiée en 1994 par Auriol et al (17).

De manière avantageuse, les anticorps bi-spécifiques utilisés dans ce procédé reconnaissent entre autres les protéines Tat, Rev du rétrovirus VIH, ainsi que des marqueurs de surface, CD3, CD4, CD8, CD19, et CD34.

Par ailleurs, la présente invention concerne un procédé de transfert d'un principe actif dans les noyaux de cellules eucaryotes choisies, caractérisé par le couplage de ce principe actif à un immunovecteur possédant à la fois la capacité à permettre l'internalisation de ce principe actif dans ces cellules eucaryotes et une affinité pour l'ADN de ces cellules, au point de le rendre apte à transporter ce principe biologiquement actif à proximité immédiate ou dans les noyaux de ces cellules

Ce principe biologiquement actif peut être couplé de façon covalente ou non covalente à l'immunovecteur.

Préférentiellement, ce procédé de transfert est caractérisé en ce que l'immunovecteur entrant dans la constitution de ce produit est choisi parmi ceux qui sont sélectionnables par un test de pénétration cellulaire comportant une première incubation de l'immunovecteur étudié en présence de cellules dans le noyau desquelles le principe actif susceptible d'être associé à l'immunovecteur doit être transporté, suivie après fixation et perméabilisation de ces cellules, d'une autre incubation avec des anticorps anti-immunovecteurs marqués, et enfin la détection à proximité immédiate du noyau ou même au sein de celui-ci de la réaction immunologique du type antigène-anticorps entre l'immunovecteur et l'anticorps anti-immunovecteur.

Dans une réalisation préférée du procédé de transfert selon l'invention, l'immunovecteur utilisé est formé d'un anticorps ayant une affinité pour un acide nucléique, ou d'un fragment de cet anticorps conservant cette affinité.

Cet immunovecteur utilisé dans ce procédé est préférentiellement choisi parmi les anticorps, de préférence des IgG monoclonales, les fragments (Fab')2 ou (Fab'), ou tous polypeptides correspondant au(x) site(s) des anticorps impliqué(s) dans la reconnaissance de l'acide nucléique correspondant.

Avantageusement, une fois le produit de couplage immunovecteur/principe actif préparé, il peut être utilisé pour le transfert intranucléaire d'autres molécules. Ainsi, au conjugué fluorescéine/immunovecteur expérimenté, on peut associer un anticorps anti-fluorescéine couplé avec une molécule tierce, et ainsi transférer ladite molécule tierce dans les noyaux des cellules De façon similaire, le conjugué biotine/immunovecteur peut permettre la liaison d'un anticorps anti-biotine ou d'avidine-streptavidine couplé avec une molécule tierce à transférer dans les noyaux.

Dans la présente invention, on a transféré dans le noyau une enzyme telle que la peroxydase de raifort, mais d'autres protéines possédant des activités biologiques variées peuvent aussi être utilisées. On a aussi utilisé la peroxydase couplée à l'immunovecteur par l'intermédiaire du glutaraldéhyde. Cependant, d'autres procédés connus dans la littérature, comme ceux décrits dans le cas des haptènes, peuvent être aussi utilisés.

Comme dans le cas des conjugués haptènes/immunovecteur, il peut être utilisé, en association avec les conjugués protéine/immunovecteurs, un anticorps anti-protéine couplé avec une molécule tierce pour le transfert intranucléaire de ladite molécule.

Dans l'invention ici décrite, bien qu'on ait transféré un polynucléotide dans le noyau, une très grande variété d'acides nucléiques possédant des activités biologiques appropriées peut être également activement transférée au niveau intranucléaire.

Ainsi un procédé de transfert de principes actifs selon l'invention permet notamment le transfert de gènes destinés à s'intégrer dans le génome des cellules cibles, notamment par recombinaison homologue plus particulièrement le transfert d'ADN "nu", ce dernier pouvant être notamment utilisé en tant qu"'ADN vaccin".

Une des techniques de recombinaison homologue possible est celle décrite par le Mouellic et al. en 1990 (18).

De récents travaux effectués par Whalen R. G. et al ont permis de montrer l'existence d'une réponse immunitaire suite à un transfert d'ADN. Ces travaux qui ont fait l'objet de la demande de brevet WO 95/11307. ont été plus particulièrement appliqués à l'expression de molécules monoclonales du type cytokine IL2 (19).

En outre, ce procédé de transfert permet d'introduire des séquences nucléotidiques impliquées dans l'immortalisation de différents types cellulaires, particulièrement les macrophages, les cellules dendritiques, les cellules B et T, et les cellules hématopoïétiques notamment d'origine humaine. A titre de séquences nucléotidiques on peut citer les séquences oncogènes ou des séquences virales associées à des phénomènes de transformation cellulaire.

Il permet également le transfert d'oligonucléotides antisens permettant l'inhibition de la synthèse protéique ou nucléotidique, par exemple dans les cellules infectables par un rétrovirus tel que VIH, ou les cellules tumorales.

Dans la présente invention, le polynucléotide a été couplé à l'immunovecteur par l'intermédiaire de la p-benzoquinone. Cependant, d'autres procédés connus dans la littérature peuvent être aussi employés.

Par ailleurs, la présente invention est aussi relative aux cellules eucaryotes contenant des molécules actives de préférence au niveau nucléaire, caractérisées en ce que lesdites molécules ne sont pas naturellement incorporables aux noyaux desdites cellules, ou ont un taux d'expression faible dans lesdites cellules. Ces molécules sont présentées dans ces cellules à proximité immédiate de leurs noyaux ou dans ceux-ci, et couplées à un immunovecteur caractérisé par son affinité pour l'ADN de ces cellules, à l'état de produit de couplage selon l'invention.

Parmi les cellules visées par la présente invention, on retrouve notamment les cellules infectables par un virus ou les cellules tumorales.

Entrent également dans le cadre de la présente invention les hybridomes produisant les anticorps selon la présente invention, tels que déposés à la CNCM le 30 juin 1995 sous les numéros I-1605, I-1606, I-1607.

En outre, l'invention est relative à une composition pharmaceutique caractérisée en ce qu'elle contient, en association avec un véhicule physiologiquement acceptable, un produit de couplage selon l'invention, dans lequel le principe biologiquement actif est un principe actif de médicament ou de vaccin et l'immunovecteur est compatible avec l'organisme de l'hôte auquel le médicament est destiné.

Entre également dans le cadre de la présente invention, l'utilisation du produit de couplage de l'invention pour l'expression dans des cellules réceptrices d'une séquence nucléotidique hétérologue par rapport à l'ADN de l'hôte.

### EXEMPLES

### I. PREPARATION D'IMMUNOVECTEURS

### A) Immunovecteurs polyclonaux

Les IgG humaines ou murines sont d'abord isolées par passage d'un mélange de sérums provenant d'individus atteints de lupus érythémateux disséminé - ou de souris lupiques (NZB x NZW)F1 - sur de la protéine A immobilisé sur Sepharose (14).

Les IgG isolées sont passées sur une colonne d'ADN immobilisé sur de la cellulose. Les anticorps spécifiques anti-ADN présents dans ces IgG sont fixés sur cet immunoadsorbant ADN-cellulose et élués avec un tampon carbonate bicarbonate de sodium 20 µM, pH 10, contenant 5 % de diméthylsulfoxide (15). On isole ainsi 1 à 2 mg d'anticorps à partir de 10 mg d'IgG Les anticorps élués sont dialysés, concentrés et conservés à +4°C jusqu'à leur utilisation.

### B) Immunovecteurs monoclonaux murins

Les splénocytes provenant de souris lupiques (NZB x NZW)F1 sont fusionnés avec le myélome X63 selon le procédé de Köhler et Milstein. Les hybridomes produits sont testés en ELISA pour la sécrétion d'IgG et pour leur activité anti-ADN. Les hybridomes sécréteurs d'IgG anti-ADN sont sous-clonés au moins deux fois et les clones demeurés doublement positifs (IgG + anti-ADN) sont cultivés en masse ou bien des ascites sont préparés à partir de ces clones chez la souris. Dans une expérience typique à partir de la rate d'une souris (NZB x NZW)F1, on a obtenu approximativement 300 puits positifs sécrétant des IgG dont 60 étaient capables de réagir avec l'ADN. Après clonage, 20 clones sécrétaient de l'IgG reconnaissant l'ADN. De ces 20 clones, approximativement la moitié sécrétaient des anticorps capables de pénétrer dans le noyau des cellules alors que les autres n'en étaient pas capables (voir C : test de pénétration intranucléaire des immunovecteurs). Les IgG monoclonales sont isolées à partir des surnageants de culture ou des liquides d'ascites par précipitation avec du sulfate d'ammonium à 45% suivi, après dialyse, par passage sur de la protéine A immobilisé sur Sepharose. Après neutralisation des IgG éluées, les préparations sont dialysées, concentrées et gardées à -20°C jusqu'à leur utilisation.

Les anticorps de souris ci-dessus évoqués seront subséquemment humanisés par l'utilisation d'une des techniques connues, par exemple celle décrite par Riechmann et al. (20)

### C) Sélection des immunovecteurs

### 1) Test de pénétration intranucléaire des immunovecteurs

Deux lignées de fibroblastes - PtK2 provenant de rein de rat kangourou et GMA-32 provenant de rein de hamster - ont été principalement utilisées. Les lames portant les fibroblastes en croissance exponentielle ensemencées à 2 x 10⁴ cellules/ml, 24 heures auparavant et mises en culture dans du milieu RPMI 1640 ou MEM (contenant 10 % du sérum de veau foetal, 2mM-L-glutamine et 1 % de pyruvate de sodium) sont incubées à 37°C dans du milieu de culture renouvelé, contenant des quantités choisies d'immunovecteur (1 à 70 µg/ml). Après 2 à 4 heures d'incubation, les cellules sont lavées avec du PBS et fixées avec soit de l'éthanol pendant 10 minutes à -20°C, soit avec 0.2 % de glutaraldehyde et 2 % de formaldehyde dans du PBS pendant 20 minutes. Après trois lavages avec du PBS, les cellules sont perméabilisées pendant 20 minutes dans du PBS contenant 0.2 % de sérum albumine bovine et 0.5 % de saponine.

Les préparations cellulaires sont alors lavées avec du PBS et incubées pendant 45 minutes à 24°C avec des anticorps de lapin ou de mouton anti-immunoglobulines de souris (ou anti-immunoglobulines humaines) marqués avec la fluorescéine ou avec la peroxydase (20 µg/ml) Après lavage, les préparations cellulaires incubées avec l'anticorps fluorescent sont examinées au microscope à fluorescence. Les préparations cellulaires incubées avec l'anticorps marqué avec la peroxydase sont d'abord incubées dans le substrat cytochimique de la peroxydase (diaminobenzidine (DAB) + H₂O₂) et, après lavage, la préparation est examinée au microscope optique (14). Le nombre de cellules positives est compté.

Tel que précédemment décrit, des thymocytes de souris ont été aussi utilisés pour tester la pénétration des immunovecteurs dans le noyau Des suspensions de thymocytes ont été préparées à partir de thymus de souris BALB/c. Les thymocytes, à une concentration de 1 x 10⁶ cellules/ml, sont incubées à 37°C pendant 3 heures dans un milieu de culture contenant des quantités croissantes d'immunovecteur (1 à 70 µg/ml). Après lavage et fixation, les lymphocytes sont traités comme les préparations de fibroblastes ci-dessus pour la détection intranucléaire des anticorps.

### 2) Test de fixation des anticorps anti-DNA sur les cellules lymphoïdes

De façon à mettre en évidence une réaction avec les membranes des cellules, 10⁶ thymocytes ou splénocytes de souris ont été incubés à froid pendant 45 minutes avec 0,1 ml des différents anticorps monoclonaux dilués dans une solution d'albumine bovine à 0,1 % contenant 0,2 % d'azide de sodium. Après lavage, les cellules sont incubées avec des anticorps anti-lgG de souris fluorescent pendant 45 minutes à froid. Après lavage, les cellules sont examinées au FACS et le nombre de cellules positives déterminé dans chaque population.

Des 20 anticorps monoclonaux examinés, approximativement la moitié sécrètent des anticorps qui sont capables de pénétrer dans le noyau des cellules alors que l'autre moitié n'en est pas capable. Une corrélation a pu être établie entre les anticorps monoclonaux pénétrant avec une grande efficacité dans le noyau des cellules (nombre de cellules marquées, dilution limite pour obtenir un marquage) et leur aptitude à marquer les thymocytes et les splénocytes.

### II PREPARATION D'IMMUNOVECTEURS PORTANT DES HAPTENES, PROTEINES OU ACIDES NUCLEIQUES

### A) Préparation de fragments F(ab')2 et Fab' d'immunovecteurs

Les fragments F(ab')2 des immunovecteurs sont préparés selon des procédés décrits impliquant une protéolyse par la pepsine suivie d'une réduction par la cystéine pour obtenir le fragment Fab' (14). Ainsi dans 5 ml de tampon citrate-acide citrique 0,1M, pH 3,5, contenant 5 mg d'immunovecteur, on ajoute 150 µg de pepsine et on incube 2 heures à 37°C. Le milieu est ajusté à pH 8 et la préparation filtrée sur une colonne de protéine A-Sepharose pour éliminer les IgG non digérées. Après dialyse contre du PBS, cette préparation de F(ab')2 est conservée à -20°C jusqu'à utilisation. Pour obtenir les fragments Fab' on ajoute de la cystéine à une concentration finale de 0.02 M dans la préparation du F(ab')2. Après 10 minutes d'incubation à 37°C, on ajoute 0.04 M d'iodoacétamide et on laisse incuber pendant 30 minutes. Cette préparation de Fab' est dialysée contre du PBS et est conservée à -20°C jusqu'à son utilisation.

### B) Immunovecteurs/haptènes

### Couplage à la biotine

Dans 0,5 ml de tampon phosphate 0,1M, pH7, contenant 1 mg d'anticorps, on ajoute 2 µl d'une solution 0.1M de d-biotine-N-hydroxysuccinimide ester dans du dimethylformamide (1 mg de l'ester actif dans 30 µl de dimethylformamide). On laisse la solution pendant 1 heure à la température du laboratoire et on dialyse contre du PBS à +4°C toute la nuit.

### Couplage à la fluorescéine

Dans 1 ml d'une solution de carbonate de sodium 0.1M contenant 1 mg d'anticorps, on ajoute 20 µl d'une solution d'isothiocyanate de fluorescéine dans le dimethylsulfoxyde (10 mg/ml). On laisse la solution 3 heures à la température du laboratoire et on dialyse contre du PBS à +4°C.

### C) immunovecteurs/protéines

### Couplage avec la peroxydase

Dix milligrammes de peroxydase sont dissous dans 0.2 ml de glutaraldehyde à 1 % dans du tampon phosphate 0.1M pH 6,8. Après incubation à la température du laboratoire pendant 18 heures, la solution est filtrée sur une colonne de Sephadex G25 (0,9 x 60 cm) équilibrée avec NaCI 0.15M pour éliminer l'excès de glutaraldehyde. A cette solution de peroxydase activée, on ajoute 1 ml d'une solution de NaCI 0.15 M contenant 5 mg d'anticorps et 0,2 ml de tampon carbonate-bicarbonate 1M pH 9.5. La solution est gardée à +4°C pendant 24 heures puis additionnée de lysine à la concentration finale de 0.1 M, puis dialysée contre PBS à 4°C

### D) Immunovecteurs/acides nucléiques

### Polynucléotides

Le polynucléotide utilisé était composé de 15 nucléotides et portait une fluorescéine en 5' et un groupement NH₂ libre en 3'. Il a été préparé selon des procédés classiques de synthèse nucléique. Ce nucléotide a été couplé à l'immunovecteur par l'intermédiaire de la p-benzoquinone (14). Dans 0,4 ml de tampon phosphate 0.1 M pH 6 contenant 1 mg d'immunovecteur (molécule entière, F(ab')2 ou Fab') on ajoute 0,1 ml d'éthanol contenant 3 mg de p-benzoquinone. Après une incubation d'une heure à la température du laboratoire la préparation est filtrée sur colonne de Sephadex G-25. La fraction contenant l'immunovecteur activé est additionnée du polynucléotide dans un rapport d'une molécule d'immunovecteur pour 4 molécules de polynucléotide, et la solution ramenée à pH 9.2 avec du tampon carbonate-bicarbonate Après 18 heures d'incubation à la température du laboratoire, la réaction est arrêtée par addition de lysine à la concentration de 0.1M finale et ensuite dialysée contre du PBS. Cette préparation est conservée à +4°C jusqu'à son utilisation

### Plasmides

Deux plasmides ont été testés, un premier portant le promoteur de la vimentine en amont du gène codant pour les antigènes T, t du SV40 (pHuVim 830 T,t) (21) et un deuxième portant le gène de la luciférase (22) sous le contrôle d'un promoteur du cytomegalovirus (pCMV-Luc) (5).

Ces plasmides sont maintenus dans la souche E. coli et sont préparés après une culture bactérienne par la méthode standard de lyse en présence de détergent et en milieu alcalin Les plasmides sont ensuite purifiés par chromatographie sur une colonne de résine (Qiagen Plasmid Kits).

Les immunovecteurs J-20.8 et F-14.6 sont utilisés pour ce travail. Ces anticorps sont préparés par la méthode de préparation d'immunovecteurs monoclonaux précédemment décrite au paragraphe I.B/ . Les anticorps sont couplés à de la poly-L-lysine à l'aide d'un agent de couplage, particulièrement un carbodiimide, tel l'EDC (1-(3-dimethylaminopropyl)-1'-ethyl carbodiimide). Dans certains cas, des IgG polyclonales sont ajoutées à l'anticorps anti-ADN dans un rapport 10 : 1 de façon à augmenter la concentration d'IgG dans le milieu et favoriser ainsi le couplage avec la polylysine.
- 2 mg d'anticorps monoclonal (J-20.8 ou F-14.6) dans 1 ml de PBS ou d'IgG polyclonales concentrées à 20 mg/ml sont dialysés toute la nuit contre un tampon MES, 10 mM, pH 5. Deux mg de poly-L-lysine (PM = 18.000) sont dissous dans 1 ml de ce même tampon puis additionnés de 0,2 mg de EDC (dans 50 µl de tampon MES) pendant 30 secondes. La solution de poly-L-lysine est alors ajoutée à la mixture anticorps/EDC et l'incubation se poursuit pendant 2 heures.
- la préparation est ensuite filtrée sur une colonne protéine A-Sépharose pour séparer l'excès de poly-L-lysine des anticorps conjugués à la polylysine qui sont élués à pH 3 dans les conditions habituelles, neutralisés et dialysés contre du PBS.

### III. EXEMPLES DE TRANSFERT DE SUBSTANCES DANS LES NOYAUX DES CELLULES PAR DES IMMUNOVECTEURS ASSOCIES A CES SUBSTANCES

### A) Transfert in vitro de la fluorescéine

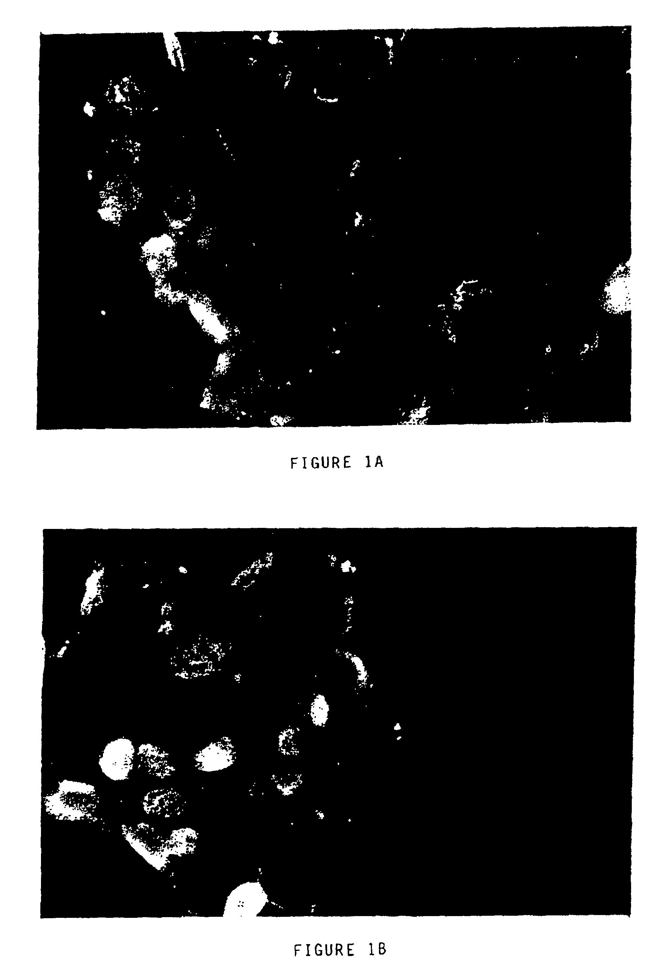

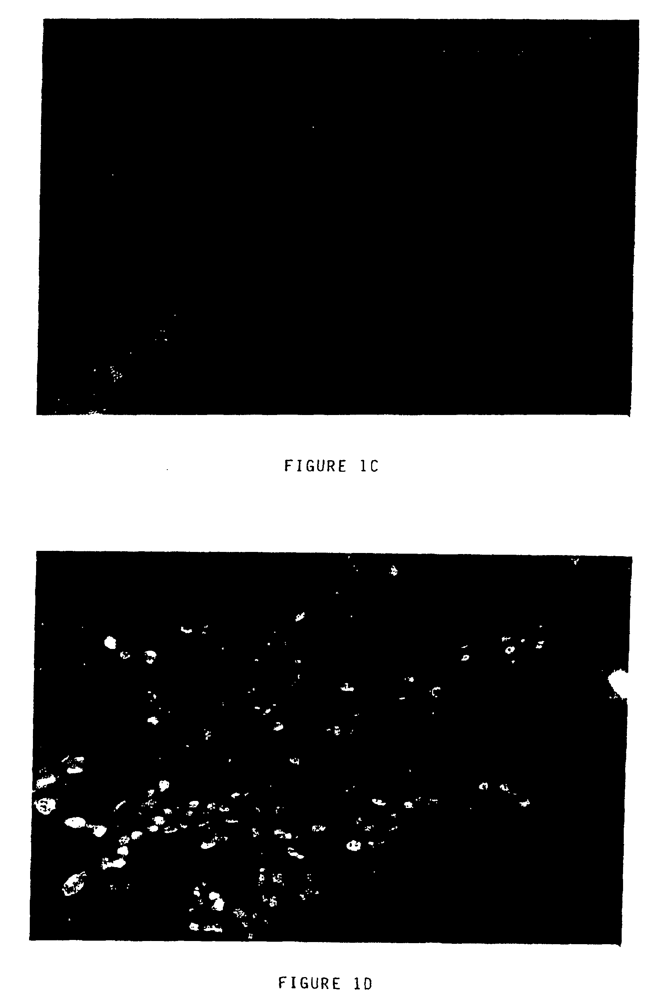

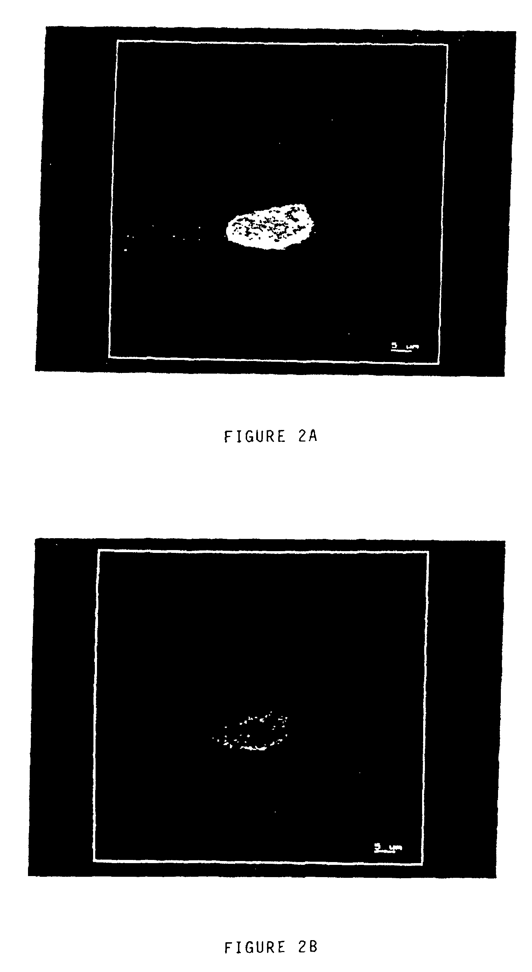

Des fibroblastes de la lignée GM A-32 en culture sur des lamelles de verre sont incubés à 37°C pendant 2 à 4 heures dans du milieu de culture RPMI contenant des quantités croissantes d'immunovecteurs monoclonaux (anticorps J-20.8 ou fragments Fab'2) marqués avec la fluorescéine. Au bout de ce temps, les cellules sont lavées et fixées comme décrit dans IC1. Après inclusion dans le milieu Mowiol, elles sont examinées au microscope en fluorescence. La presque totalité des noyaux des fibroblastes montre un marquage fluorescent. Par contre, les noyaux de fibroblastes incubés avec un anticorps monoclonal témoin lg 2a sans activité anti-ADN, qui ne pénètre pas dans les noyaux, ne présentent pas de fluorescence (Figures 1 et 2)

### B) Transfert in vivo de la fluorescéine dans les lymphocytes périphériques de la souris

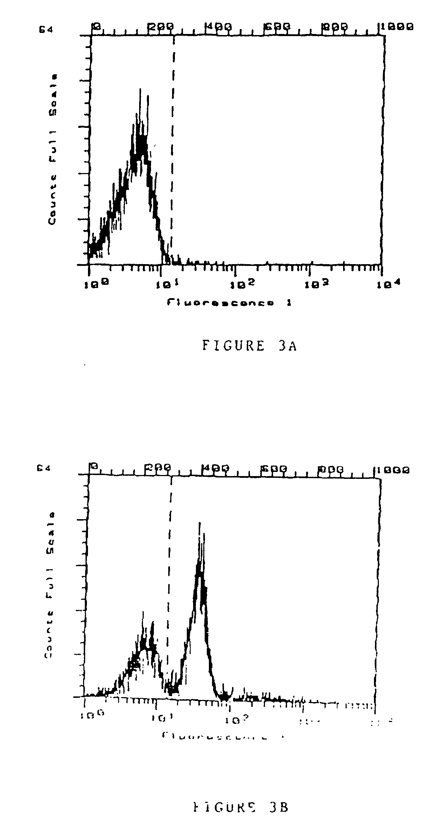

Un mg d'immunovecteur (anticorps monoclonal C-2.1 ou F-4.1 ou d'anticorps témoin (anticorps monoclonal G-14) marqué avec la fluorescéine est injecté à deux souris à raison de 0,2 ml par voie intraveineuse et 0,3 ml par voie intrapéritonéale. Après 5 heures les souris sont saignées et sacrifiées et les lymphocytes du sang circulant sont analysés par FACS. On note que 60% des lymphocytes du sang périphérique provenant de la souris injectée avec l'immunovecteur sont fluorescents alors qu'aucun des lymphocytes de l'animal témoin ne le sont (Figure 3). L'examen au microscope montre une fluorescence au niveau des noyaux dans la majorité des cellules.

### C) Transfert de la biotine

Des fibroblastes de la lignée PtK2 (10⁵/ml) mis en culture 24 heures auparavant sont incubés dans un milieu de culture RPMI complet avec des IgG polyclonales anti-DNA humaines marquées avec la biotine en quantités croissantes (5-100 µg). Après 3 heures, les cellules sont lavées, fixées et perméabilisées comme décrit dans IC. Les cellules sont ensuite incubées avec du RPMI contenant 1 µg/ml de streptavidine marquée à la peroxydase. Après une heure, les cellules sont lavées trois fois avec du PBS et la peroxydase associée aux cellules est révélée en utilisant le milieu DAB + H₂O₂. Les préparations sont incluses dans du Mowiol et examinées au microscope optique Un grand nombre de noyaux des fibroblastes incubés avec l'IgG anti-ADN sont positifs alors que les cellules incubées avec des IgG provenant d'individus normaux et marquées avec la biotine, sont négatives.

### D) Transfert de la peroxydase

Dans les conditions définies en paragraphe IIIC, les fibroblastes PtK2 sont incubés avec des quantités croissantes de fragments Fab' d'un immunovecteur (anticorps J-20.8) marqué avec la peroxydase. Après 3 heures, les cellules sont lavées trois fois avec du PBS et fixées pendant 20 minutes avec 0.2 % de glutaraldehyde et 2 % de formaldehyde dans du PBS. Après lavage, on révèle l'activité de la peroxydase par le test coloré DAB + H₂0₂ et on examine les préparations au microscope optique. Une grande proportion de noyaux des fibroblastes incubés avec les fragments Fab' de l'anticorps J-20.8 sont positifs pour la peroxydase, alors que ceux incubés avec l'anticorps contrôle 48.9 sont négatifs.

### E) Transfert de polynucléotide marqué à la fluorescéine

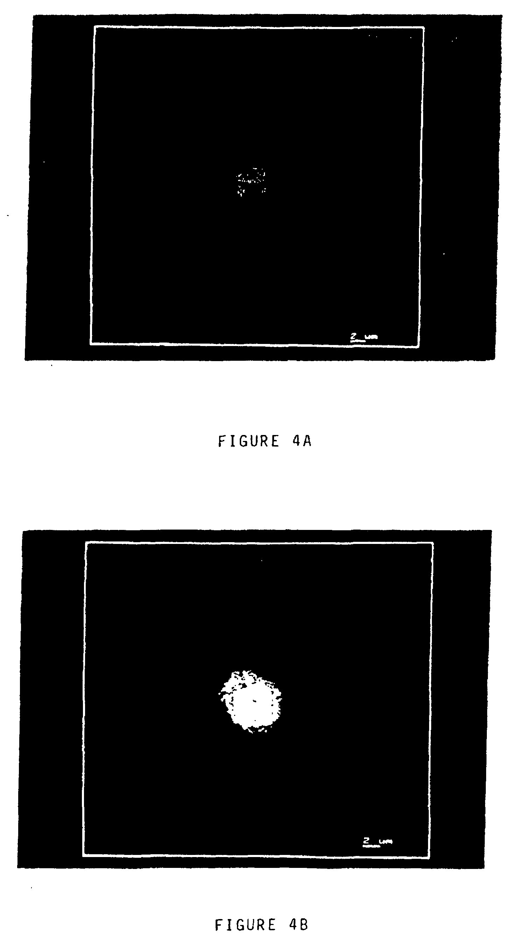

3 x 10⁶ splénocytes, préparés à partir de la rate de souris BALB/c, sont incubés dans 1 ml de RPMI contenant 40 µg/ml d'immunovecteur (J-20.8) ou de son fragment Fab' couplé de manière covalente au polynucléotide Après trois heures d'incubation à 37°C, les cellules sont lavées avec du PBS. fixées en 4% de paraformaldehyde et examinées au microscope. Huit à 10 % des cellules montrent une fluorescence intranucléaire (Figure 4).

### F) Transfert de plasmide

L'efficacité de transfection a été évaluée par la mise en évidence de la synthèse des protéines codées par ces gènes, soit à l'aide d'anticorps anti-antigène T couplés à la péroxydase, soit par un dosage luminométrique de l'activité de la luciférase sur son substrat la luciférine.

Les cellules utilisées sont des fibroblastes de la lignée GMA-32 et des cellules de carcinome Hep 2. Elles sont cultivées dans un milieu complet (milieu RPMI 1640 contenant 10% de sérum de veau foetal, 2mM L-glutamine, 1% de pyruvate de sodium et des antibiotiques), à 37°C de 5% de CO₂.

*Plasmide pHuVim 830 T,t* : Les cellules Hep2 sont ensemencées la veille à raison de 2 x 10⁴ cellules dans 0,5 ml de milieu complet par puits d'une plaque de 24 puits. Pour la transfection, le milieu est retiré et remplacé par 0,3 ml de milieu complet contenant 20 µg d'anticorps-poly-L-lysine et 2 µg de plasmide, ou 20 µg d'anticorps natifs et 2 µg de plasmide ou 2µg de plasmide seul. Après 6 heures, le milieu est changé et la culture est poursuivie en changeant le milieu tous les deux jours et en dédoublant les cellules si nécessaires. L'efficacité de la transfection est testée à des temps variables.

*Plasmide pCMV-Luc* : les cellules GMA-32 sont ensemencées la veille à raison de 7 à 10 x 10⁴ cellules / 0,5 ml de milieu complet par puits d'une plaque de 24 puits de milieu de culture complet. Pour la transfection, le milieu est retiré et remplacé par 0,5 ml de milieu complet contenant 8 µg de J-20.8/polysine ou de F-14 6/polylysine, ou 20 µg de J-20 8 IgG polyclonale et 2 µg de plasmide ou 2 µg de plasmide seul. Après 6 heures, le milieu est changé. L'efficacité de la transfection et testée 24 heures après le début de la transfection.

### Contrôle de la transfection

*Plasmide pHuVim 830 T,t* : la synthèse de l'antigène T dans le noyau des cellules transfectées est mise en évidence par une méthode immunocytochimique Les cellules sont lavées 3 fois avec du PBS puis fixées 10 minutes dans le méthanol à -20°C. Elles sont ensuite incubées avec l'anticorps anti-antigène T couplé à la peroxydase pendant 1 heure. Après lavage, la péroxydase est révélée par le mélange DAB + H₂O₂. Dans le puits incubé avec le complexe J-20.8-polylysine et plasmide, des cellules isolées et quelques foyers de cellules ont un noyau fortement coloré en brun après 48 heures et après 2 semaines Le témoin avec l'anticorps natif ou le plasmide seul est négatif.

*Plasmide pCMV-Luc* : l'efficacité de la transfection est mise en évidence par la synthèse de luciférase détectable dans les lysats des cellules transfectées. Cette enzyme catalyse l'oxydation de la luciférine qui se traduit par un produit détectable dans un luminomètre. Après la culture, les cellules sont lavées dans le PBS puis lysées dans un tampon Trisphosphate, 25 mM, pH 7,8, contenant 8 mM MgCl₂, 1 mM DTT, 1% Triton X100, 1% BSA et 15% glycérol. Le lysat est dosé dans un luminomètre par addition automatique d'une solution de luciférine (0,25 mM) et d'ATP (1 mM). Un aliquot du même lysat est dosé quant à sa concentration en protéines par un réactif de Coomassie (Bio-Rad Protein Assay). Les résultats sont exprimés en unités (RLU) par mg de protéines. Comme le montre le tableau, un transfert de gènes a lieu en présence des préparations anticorps J-20.8/polylysine et F-14.6/polylysine alors que les IgG / polylysine n'ont pas d'effet.

L'efficacité de transfection pour un même rapport anticorps/plasmide est 10 fois plus élevée avec la préparation J-20.8 qu'avec le F-14.6. De plus, l'addition d'IgG polyclonales concentrées pendant le couplage à la polylysine semble augmenter l'efficacité de transfection puisque 2 µg de J-20.8 (complexe 20 : 0,5) de la préparation J-20.8-lgG/polylysine donnent des résultats du même ordre de grandeur que 8 µg (complexe 8 : 0,5) de la préparation J-20.8/polylysine.

L'ensemble des résultats obtenus est résumé dans le tableau suivant :

| Immunovecteur | Rapport anticorps/plasmide µg/µg | Dosage RLU/mg x 10⁴ |
|---|---|---|
| J-20.8-IgG /polylysine | 20 : 0,5 | 5,8 |
| | 20 : 0,5 | 63,00 |
| J-20.8/ polylysine | 8 . 2 | 2,00 |
| | 8 : 1 | 15,00 |
| | 8 : 1 | 38,00 |
| | 8 : 0,5 | 13,00 |
| F-14.6/ polylysine | 8 · 0,5 | 1,3 |
| | | |
| IgG/polylysine | 20 : 0,5 | < 0,1 |
| | 20 : 0,5 | <0,1 |
| Plasmide seul (µg) | 2 | < 0,1 |
| | 1 | < 0,1 |
| | 0,5 | < 0,1 |

Dans ce qui précède, les immunovecteurs préférés étaient essentiellement formés par des anticorps anti-ADN ou des fragments de ces anticorps, sous réserve que ces fragments retiennent le site de reconnaissance pour l'ADN entier. II va naturellement de soi que les immunovecteurs utilisables dans le cadre de l'invention pourraient être constitués de toute autre façon, dès lors qu'ils permettraient également le transport du principe biologiquement actif qui lui serait associé à travers les membranes de ces cellules et leur cytoplasme et son transfert à proximité du noyau des cellules, ou même à l'intérieur de ce noyau.

A titre d'exemples de tels immunovecteurs, on peut citer des conjugués entre une protéine nucléaire, par exemple un histone, une protéine hnRNP, une polymérase ou un facteur associé à cette polymérase, et le produit actif, cette protéine nucléaire étant elle-même ( sauf à ce qu'elle soit à elle seule capable d'assurer l'internalisation et le transfert d'un principe biologiquement actif dans le noyau des cellules) conjuguée à l'anticorps anti-récepteur membranaire de la cellule ou à toute autre molécule permettant l'internatilisation dans la cellule du conjugué ainsi produit. Dès lors que ce conjugué est apte à diffuser jusque dans le noyau des cellules et que par ailleurs il pourrait à son tour transporter et transférer, comme cela a été défini plus haut, un principe biologiquement actif qui serait couplé à ce conjugué, il constitue un immunovecteur entrant dans le cadre de la présente invention.

Les techniques de sélection qui ont été décrites, plus haut, pour le choix d'immunovecteurs efficaces pour le transfert d'un principe actif dans le noyau des cellules sont tout autant applicables à la sélection des conjugués susmentionnés.

L'homme du métier comprendra qu'un critère de choix supplémentaire pourrait, pour au moins certains des conjugués utilisés, résider dans l'absence d'une intervention indésirable de la protéine nucléaire qu'il contient avec le fonctionnement de la cellule. Il est d'ailleurs à noter que seule la partie de la protéine intranucléaire portant son site de reconnaissance de l'ADN correspondant est indispensable à la réalisation conforme à l'invention.

### LEGENDE DES FIGURES

**FIGURE 1** : Transfert "in vitro" de la fluorescéine.
   - Marquage des noyaux des fibroblastes GMA 32 par un immunovecteur marqué à la fluorescéine (anticorps J-20.8) en A et B (grossissement x 100).
   - C : Absence de marquage avec l'anticorps contrôle fluorescent (x 100).
   - D : Un autre champ vu au faible grossissement (x 40).
**FIGURE 2** : Même préparation que celle de la Figure 1A analysée en microscope confocale. Les fibroblastes GMA 32 sont marqués essentiellement dans le noyau. Sur la figure 2A, on peut noter une fluorescence totale. La figure 2B correspond à l'analyse de l'intensité de fluorescence.
**FIGURE 3** .Transfert de la fluorescéine "in vivo.
   Analyse au FACS des cellules du sang périphérique des souris injectées 5 heures auparavant sur la figure 3A, l'anticorps contrôle fluorescent et sur la figure 3B avec un immunovecteur fluorescent l'anticorps (J-20.8). Histogrammes représentant en abscisse, l'intensité de fluorescence (en unité arbitraire) et en ordonnée, le nombre de cellules.
**FIGURE 4** : Analyse des cellules du sang périphérique (de la figure 3) en microscopie confocale. Sur la figure 4A, on peut noter une fluorescence totale. La figure 4B correspond à l'analyse de l'intensité de fluorescence.

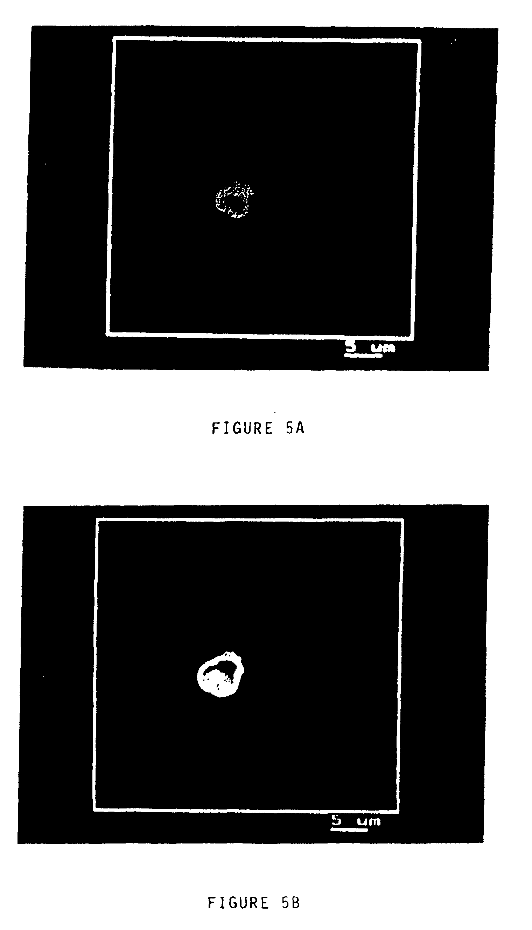

**FIGURE 5** : Transfert "in vitro" d'un nucléotide.
   Examen en microscopie confocale d'un splénocyte de souris dans lequel a pénétré le fragment Fab' d'un immunovecteur (anticorps J-20.8) couplé à un nucléotide fluorescent. Sur la figure 5A, on observe une fluorescence totale. La figure 5B correspond à l'analyse de l'intensité de fluorescence.

### REFERENCES

(1) "Non-viral therapy", Current Opinion on Biotechnology. Vol. 5. p 626-636, (1994), Ledley F.D.
(2) T.D. McKee, M.E. DeRome, G.Y. WU and M.A. Findeis. Preparation of asioloorosolucoid-polylysine conjugates. Bioconjugate Chem., 5:306 (1994)
(3) Receptor-mediated endocytosis and nuclear transport of transfecting DNA construct. A.A. Rosenkrank, S.V. Yachmenev, D. A. Jans, N V. Serebryakova, V.I. Murav'ev, R. Peters and A.S. Sobolev. Exp. Cell Res., 199:323 (1992).
(4) Receptor-mediated endocytosis of transferrin-polycation conjuguates an efficient way to introduce DNA into hematopoietic cell M. Zenke, P Steinlein, E. Wagner, M. Cotte, H. Beug, and M.L. Birnstiel. Proc. Natl. Acad. Sci. USA, 87:3655 (1990)
(5) J.R. Merwin, E.P. Carmichael, G.S. Noell, M.E. DeRome, W.L. Thomas, N. Robert, G. Spitalny and H.C. Chiou. CD5-mediated specific delivery of DNA to T lyphocytes: compartmentalization augmented by adenovirus. J. Immunol. Methods, 186:257 (1995).
(6) E. Wagner, K. Zatloukal, M. Cotten, H. Kirlappos, K. Mechtler, D.T. Curiel and M.L. Birnstiel. Coupling of adenovirus to transferrin-polylysine/DNA complexes enhances receptor-mediated gene delivery and expression of transfected genes. Proc. Natl. Acad. Sci USA 89 6099 (1992).
(7) "Antibody to nuclear ribonucleoptotein penetrates live human mononuclear cells through Fc receptors", Nature, Vol. 271 (1978), Donarto Alarcon-Segovia et al.
(8) "Antibody penetration into living cells", The Journal of Immunology, Vol. 122, (1979), Donarto Alarcon-Segovia et al.
(9) "V_{H} gene analysis of spontaneously activated B cells in adult MRL-lpr/lpr mice", The Journal of Immunology, Vol. 147, pages 1504-1511, (1991), Mary H. Foster et al.
(10) "Murine Monoclonal Anti-DBA Antibodies Penetrate Cells, Bind to Nuclei, and Induce Glomerular Proliferation and Proteinuria In Vivo", J. Am. Soc. Nephrol., Vol.2, p. 1345-1354 (1992), Demetrios Vlahakos et al.
(11) "Monoclonal Murine anti-DNA antibody interacts with living mononuclear cells", Arthritis and Rheumatism, Vol. 30 No. 6, p. 669-678, (1987), Okudaira et al.
(12) "Cross-reactions of anti-DNA autoantibodies with cell surface proteins", Eur. J. Immunol. Vol. 23, p. 383-390, (1993). Eyal Raz et al.
(13) "Life Science & Analytical Research Products": Pierce Interchim
(14) "Techniques immunoenzymatiques", Editions Inserm, (1987), Ternynck T. and Avrameas S.
(15) "DNA affinity column chromatography: application in the isolation of distinct antibody populations SLE sera., Clin. Exp. Immunol. Vol. 62, pp. 321-328, (1985) Kubota et al.
(16) "An antibody chimera technique applied to enzyme immunoassay for human alpha-1-fetoprotein with monoclonal and polyclonal antibodies", J Immunol. Methods, (1984) Vol. 66, p. 179-185. Porstmann B et al
(17) "Development of a bispecific monoclonal antibody for use in molecular hybridization, J Immunol. Methods, ((1994), Vol. 169, p. 123-133, Auriol J. et al.
(18) "Targeted replacement of the homeobox gene Hox-31 by the Escherichia coli lacZ in mouse chimeric embryos", Genetics, Proc. Natl Acad. Sci. USA, (1990), Vol. 87, p. 4712-4716, Le Mouellic et al.
(19) "DNA-mediated immunization and the energetic immune response to hepatitis B surface antigen", Clin. Immunology and Immunopathology, (1995), Vol 75, p 1-12, Whalen R. G. et al.
(20) "Rehasping human antibodies for therapy", Nature, (1988). Vol 332. p. 323-327, Riechmann et al.
(21) B. Schwartz, P. Vicart, C. Delouis and D. Paulin, Mammalian cell lines can be efficently established *in vitro* upon expression of the SV40 large T antigen driven by a promoter sequence derived from the human vimentin gene. Biol. Cell., 72:7 (1991)
(22) S.K. Nordeen. Luciferase reporter gene vectors for analysis of promoters and enhancers. Bio Techniques, 6:454 (1988).

## Revendications

1. Produit de couplage entre un principe biologiquement actif et un immunovecteur ledit produit de couplage étant caractérisé à la fois par la capacité de l'immunovecteur à permettre l'internalisation dans des cellules eucaryotes du principe biologiquement actif, et par l'affinité de l'immunovecteur pour l'ADN de ces cellules, au point de rendre ledit immunovecteur apte à transférer le principe biologiquement actif à proximité immédiate des noyaux de ces cellules ou dans ces noyaux.

2. Produit selon la revendication 1, **caractérisé en ce que** le couplage entre le principe biologiquement actif et l'immunovecteur met en oeuvre une liaison covalente ou non-covalente.

3. Produit selon la revendication 1 ou la revendication 2, **caractérisé en ce que** l'immunovecteur entrant dans la constitution de ce produit est choisi parmi ceux qui sont sélectionnables par un test de pénétration cellulaire comportant une première incubation de l'immunovecteur étudié en présence de cellules dans le noyau desquelles le principe actif susceptible d'être associé à l'immunovecteur doit être transféré, suivie, après fixation et perméabilisation de ces cellules, d'une autre incubation avec des anticorps anti-immunovecteurs marqués, et enfin d'une détection à proximité immédiate du noyau ou même au sein de celui-ci de la réaction immunologique du type antigène-anticorps entre l'immunovecteur et l'anticorps anti-immunovecteur.

4. Produit selon l'une quelconque des revendications 1 à 3, **caractérisé en ce que** l'immunovecteur est formé d'un anticorps ayant une affinité pour un acide nucléique ou un fragment de cet anticorps conservant cette affinité.

5. Produit selon la revendication 4, **caractérisé en ce que** l'immunovecteur est choisi parmi les IgG monoclonales, les fragments (Fab')2 ou (Fab'), ou tous polypeptides correspondant au(x) site(s) des anticorps impliqué(s) dans la reconnaissance de l'acide nucléique correspondant.

6. Produit selon la revendication 4 ou 5, **caractérisé en ce que** les immunovecteurs sont des immunoglobulines, notamment des IgG porteuses d'activité anti-ADN provenant d'individus normaux.

7. Produit selon la revendication 4 ou 5, **caractérisé en ce que** les immunovecteurs sont des immunoglobulines notamment des IgG porteuses d'activité anti-ADN, provenant d'individus présentant des syndromes autoimmuns, plus particulièrement des syndromes de lupus erythémateux disséminé.

8. Produit selon l'une quelconque des revendications 4 à 7, **caractérisé en ce qu'**il s'agit d'un anticorps bi-spédfique reconnaissant d'une part l'ADN et d'autre par une protéine telle que Tat, Rev du rétrovirus VIH, et, d'autre part, des marqueurs de surface tels que CD3, CD4, CD8, CD19, et CD34.

9. Produit selon l'une quelconque des revendications 1 à 8, **caractérisé en ce que** le principe actif couplé à l'immunovecteur est une molécule choisie parmi des acides nucléiques, des protéines ou des haptènes.

10. Produit selon l'une, quelconque des revendications 1 à 8, **caractérisé en ce que** le principe actif couplé à l'immunovecteur est constitué par un plasmide permettant l'expression d'une protéine codée par un gène contenu dans ledit plasmide.

11. Produit selon la revendication 10, **caractérisé en ce que** lorsque l'immunovecteur est un anticorps ou fragment d'anticorps, ce dernier est préalablement couplé à un agent capable d'induire un effet de compactage sur l'ADN, cet agent étant de préférence le polylysine.

12. Produit selon l'une quelconque des revendications 1 à 8, **caractérisé en ce que** le principe actif est constitué par un gène destiné à s'intégrer dans le génome des cellules cibles, notamment par recombinaison homologue, plus particulièrement un ADN "nu" contenant une séquence d'acide nucléique codant pour un polypeptide originaire de cellules bactériennes ou eucaryotes, de cellules fongiques, ou de virus, ce polypeptide ayant des propriétés vaccinantes.

13. Produit selon l'une quelconque des revendications 1 à 8, **caractérisé. en ce que** le principe actif permet l'immortalisation de types cellulaires choisis, particulièrement les macrophages, les cellules dendritiques, les cellules B et T, et les cellules hématopoiétiques, notamment d'origine humaine.

14. Produit selon l'une quelconque des revendications 1 à 8, **caractérisé en ce que** le principe actif est un oligonucléotide antisens permettant l'inhibition de la synthèse protéique ou nucléotidique, par exemple dans les cellules affectées par un rétrovirus VIH, ou dans des cellules tumorales.

15. Cellules eucaryotes contenant des molécules actives de préférence au niveau nucléaire, **caractérisées en ce que** lesdites molécules ne sont pas naturellement incorporables aux noyaux desdites cellules, ou ont un taux d'expression faible dans lesdites cellules, lesdites molécules étant présentées dans ces cellules à proximité immédiate de leurs noyaux ou dans ceux-ci, et couplées à un immunovecteur **caractérisé par** son affinité pour l'ADN de ces cellules, à l'état de produit selon l'une quelconque des revendications 1 à 12.

16. Cellules selon la revendication 15, **caractérisées en ce qu'**il s'agit de cellules infectables par un virus ou de cellules tumorales.

17. Hybridomes déposés à la CNCM le 30 juin 1995 sous les numéros d'enregistrement :
I-1605
I-1606
I-1607.

18. Procédé de transfert in vitro d'un Principe actif dans les noyaux de cellules eucaryotes choisies, **caractérisé par** le couplage de ce principe actif à un immunovecteur possédant à la fois la capacité à permettre l'intemalisation de ce principe actif dans ces cellules eucaryotes et une affinité pour l'ADN de ces cellules, au point de le rendre apte à transporter ce principe biologiquement actif à proximité immédiate ou dans les noyaux de ces cellules.

19. Procédé selon la revendication 18, **caractérisé en ce que** le principe biologiquement actif est couplé de façon covalente ou non covalente à l'immunovecteur.

20. Procédé selon la revendication 18 ou 19, **caractérisé en ce que** l'immunovecteur entrant dans la constitution de ce produit est choisi parmi ceux qui sont sélectionnables par un test de pénétration cellulaire comportant une première incubation de l'immunovecteur étudié en présence de cellules dans le noyau desquelles le principe actif susceptible d'être associé à l'immunovecteur doit être transporté, suivie après fixation et perméabilisation de ces cellules, d'une autre incubation avec des anticorps anti-immunovecteurs marqués, et enfin d'une détection à proximité immédiate du noyau ou même au sein de celui-ci de la réaction immunologique du type antigène-anticorps entre l'immunovecteur et l'anticorps anti-immunovecteur.

21. Procédé selon la revendication 18, **caractérisé en ce que** l'immunovecteur est formé d'un anticorps ayant une affinité pour un acide nucléique ou un fragment de ce anticorps conservant cette affinité.

22. Procédé selon la revendication 21, **caractérisé en ce que** l'immunovecteur est choisi parmi les anticorps, de préférence des IgG monoclonales, les fragments (Fab')2 ou (Fab'), ou tous polypeptides correspondant au(x) site(s) des anticorps impliqué(s) dans la reconnaissance de l'acide nucléique correspondant.

23. Composition pharmaceutique **caractérisée en ce qu'**elle contient, en association avec un véhicule physiologiquement acceptable, un produit conforme à l'une quelconque des revendications 1 à 9, dans lequel le principe biologiquement actif est un principe actif de médicament ou de vaccin et l'immunovecteur est compatible avec l'organisme de l'hôte auquel le médicament est destiné.

24. Utilisation du produit selon la revendication 1 pour exprimer in vitro dans des cellules réceptrices une séquence nucléotidique hétérologue par rapport à l'ADN de l'hôte.

## Patentansprüche

1. Kupplungsprodukt zwischen einem biologischen Wirkstoff und einem Immunvektor, wobei das Kupplungsprodukt gleichzeitig **gekennzeichnet ist durch** die Fähigkeit des Immunvektors die Einbringung des biologischen Wirkstoffs in eukaryotische Zellen zu ermöglichen, und **durch** die Affinität des Immunvektors zur DNA dieser Zellen, und zwar in einem solchen Maß, dass der Immunvektor in die Lage versetzt wird, den biologischen Wirkstoff in die unmittelbare Nähe der Kerne dieser Zellen oder in diese Kerne zu transportieren.

2. Produkt nach Anspruch 1, **dadurch gekennzeichnet, dass** die Kupplung zwischen dem biologischen Wirkstoff und dem Immunvektor eine kovalente oder nicht-kovalente Bindung schafft.

3. Produkt nach Anspruch 1 oder Anspruch 2, **dadurch gekennzeichnet, dass** der Immunvektor, der an der Bildung dieses Produkts beteiligt ist, aus den Immunvektoren ausgewählt ist, die durch einen Zelldurchdringungstest auswählbar sind, der umfasst eine erste Inkubation des untersuchten Immunvektors in Gegenwart von Zellen, in deren Kerne der Wirkstoff, der an den Immunvektor assoziiert werden kann, übertragen werden muss, gefolgt von einer weiteren Inkubation mit gegen Immunvektoren gerichteten markierten Antikörpern nach Fixierung und Permeabilisierung dieser Zellen und schließlich von einem Nachweis der immunologischen Reaktion vom Typ Antigen-Antikörper zwischen dem Immunvektor und dem gegen einen Immunvektor gerichteten Antikörper in unmittelbarer Nähe des Kerns oder sogar im Inneren desselben.

4. Produkt nach einem der Ansprüche 1 bis 3, **dadurch gekennzeichnet, dass** der Immunvektor aus einem Antikörper gebildet ist, der eine Affinität zu einer Nucleinsäure besitzt, oder aus einem Fragment dieses Antikörpers, das diese Affinität beibehält.

5. Produkt nach Anspruch 4, **dadurch gekennzeichnet, dass** der Immunvektor ausgewählt ist aus monoclonalen IgG, den Fragmenten (Fab')2 oder (Fab') oder allen Polypeptiden, die der/den Stelle(n) der Antikörper entsprechen, die an der Erkennung der entsprechenden Nucleinsäure beteiligt ist/sind.

6. Produkt nach Anspruch 4 oder 5, **dadurch gekennzeichnet, dass** die Immunvektoren Immunglobuline sind, insbesondere von normalen Personen stammende IgG mit anti-DNA-Aktivität.

7. Produkt nach Anspruch 4 oder 5, **dadurch gekennzeichnet, dass** die Immunvektoren Immunglobuline, insbesondere IgG mit anti-DNA-Aktivität sind, die von Personen stammen, die Autoimmunsyndrome aufweisen, noch spezifischer Syndrome von disseminiertem Lupus erythematodes.

8. Produkt nach einem der Ansprüche 4 bis 7, **dadurch gekennzeichnet, dass** es sich um einen bispezifischen Antikörper handelt, der einerseits die DNA und andererseits ein Protein wie z.B. Tat, Rev des Retrovirus HIV, und andererseits Oberflächenmarker wie CD3, CD4, CD8, CD19 und CD34 erkennt.

9. Produkt nach einem der Ansprüche 1 bis 8, **dadurch gekennzeichnet, dass** der Wirkstoff, der an einen Immunvektor gekoppelt ist, ein Molekül ist, ausgewählt aus den Nucleinsäuren, den Proteinen oder den Haptenen.

10. Produkt nach einem der Ansprüche 1 bis 8, **dadurch gekennzeichnet, dass** der Wirkstoff, der an den Immunvektor gekoppelt ist, aus einem Plasmid besteht, das die Expression eines Proteins erlaubt, das durch ein in dem Plasmid enhaltenen Gen codiert wird.

11. Produkt nach Anspruch 10, **dadurch gekennzeichnet, dass**, wenn der Immunvektor ein Antikörper oder ein Fragment eines Antikörpers ist, dieser/dieses vorher an ein Mittel gekoppelt wird, das einen Verdichtungseffekt auf die DNA induzieren kann, wobei dieses Mittel vorzugsweise Polylysin ist.

12. Produkt nach einem der Ansprüche 1 bis 8, **dadurch gekennzeichnet, dass** der Wirkstoff aus einem Gen besteht, das dazu bestimmt ist, sich in das Genom der Zielzellen zu integrieren, insbesondere durch homologe Rekombination, noch spezieller eine "nackte" DNA, die eine Nucleinsäuresequenz enthält, die ein Polypeptid codiert, das aus Bakterienzellen oder eukaryotischen Zellen, Pilzzellen oder aus Viren stammt, wobei das Polypeptid Impfeigenschaften besitzt.

13. Produkt nach einem der Ansprüche 1 bis 8, **dadurch gekennzeichnet, dass** der Wirkstoff die Immortalisierung der ausgewählten Zelltypen erlaubt, insbesondere der Makrophagen, der dendritischen Zellen, der B- und T-Zellen und hämatopoetischer Zellen, insbesondere solcher, die vom Menschen stammen.

14. Produkt nach einem der Ansprüche 1 bis 8, **dadurch gekennzeichnet, dass** der Wirkstoff ein Antisense-Oligonucleotid ist, das die Inhibierung der Protein- oder Nucleotidsynthese erlaubt, z.B. in von einem Retrovirus HIV befallenen Zellen oder in Tumorzellen.

15. Eukaryotische Zellen, die aktive Moleküle enthalten, vorzugsweise auf nukleärem Niveau, **dadurch gekennzeichnet, dass** die Moleküle nicht natürlicherweise in die Kerne dieser Zellen eingebracht werden können, oder in diesen Zellen eine schwache Expressionsrate haben, wobei die Moleküle in diesen Zellen in unmittelbarer Nähe der Kerne dieser Zellen oder in diesen gezeigt werden und an einen Immunvektor gekoppelt werden, der durch seine Affinität zur DNA dieser Zellen gekennzeichnet ist, im Zustand des Produkts nach einem der Ansprüche 1 bis 12.

16. Zellen nach Anspruch 15, **dadurch gekennzeichnet, dass** es sich um durch einen Virus infizierbare Zellen oder um Tumorzellen handelt.

17. Hybridome, am 30. Juni 1995 unter den Hinterlegungsnummem:
I-1605
I-1606
I-1607
bei der CNCM hinterlegt.

18. Verfahren zum in vitro Transport eines Wirkstoffs in die Kerne ausgewählter eukaryotischer Zellen, **gekennzeichnet durch** die Kopplung des Wirkstoffs an einen Immunvektor, der gleichzeitig die Fähigkeit besitzt, die Einbringung dieses Wirkstoffes in diese eukaryotischen Zellen zu ermöglichen, und eine Affinität zur DNA dieser Zellen hat, und zwar in einem solchen Maß, dass er in die Lage versetzt wird, diesen biologischen Wirkstoff in die unmittelbare Nähe oder in die Kerne dieser Zellen zu transportieren.

19. Verfahren nach Anspruch 18, **dadurch gekennzeichnet, dass** der biologische Wirkstoff kovalent oder nicht-kovalent an den Immunvektor gekoppelt ist.

20. Verfahren nach Anspruch 18 oder 19, **dadurch gekennzeichnet, dass** der Immunvektor, der an der Bildung dieses Produkts beteiligt ist, aus den Immunvektoren ausgewählt ist, die durch einen Zelldurchdringungstest auswählbar sind, der umfasst eine erste Inkubation des untersuchten Immunvektors, in Gegenwart von Zellen, in deren Kerne der Wirkstoff, der an den Immunvektor assoziiert werden kann, übertragen werden muss, gefolgt von einer weiteren Inkubation mit gegen Immunvektoren gerichteten markierten Antikörpern nach Fixierung und Permeabilisierung dieser Zellen, und schließlich von einem Nachweis der immunologischen Reaktion vom Typ Antigen-Antikörper zwischen dem Immunvektor und dem gegen einen Immunvektor gerichteten Antikörper in unmittelbarer Nähe des Kerns oder sogar im Inneren desselben.

21. Verfahren nach Anspruch 18, **dadurch gekennzeichnet, dass** der Immunvektor aus einem Antikörper gebildet ist, der eine Affinität zu einer Nucleinsäure besitzt, oder aus einem Fragment dieses Antikörpers, das diese Affinität beibehält.

22. Verfahren nach Anspruch 21, **dadurch gekennzeichnet, dass** der Immunvektor ausgewählt ist aus den Antikörpern, vorzugsweise den monoclonalen IgG, den (Fab')2 oder (Fab') Fragmenten, oder allen Polypeptiden, die der/den Stelle(n) des Antikörpers entsprechen, die an der Erkennung der entsprechenden Nucleinsäure beteiligt ist/sind.

23. Arzneimittel, **dadurch gekennzeichnet, dass** es zusammen mit einem physiologisch verträglichen Träger ein Produkt nach einem der Ansprüche 1 bis 9 enthält, in dem der biologische Wirkstoff ein Medikamenten- oder Impfwirkstoff ist und der Immunvektor mit dem Wirtsorganismus kompatibel ist, für den das Medikament bestimmt ist.

24. Verwendung nach Anspruch 1 zur in vitro Expression einer zur Wirts-DNA heterologen Nucleotidsequenz in Rezeptorzellen.

## Claims

1. A coupling product between a biologically active principle and an immunovector, said coupling product being characterized both by the capacity of the immunovector to allow internalization of the biologically active principle into eukaryotic cells, and by affinity of the immunovector for the DNA of said cells, so as to render said immunovector capable of transferring the biologically active principle to the immediate proximity of the nucleus of said cells or into said nuclei.

2. A product according to claim 1, **characterized in that** the coupling between the biologically active principle and the immunovector employs a covalent or noncovalent bond.

3. A product according to claim 1 or claim 2, **characterized in that** the immunovector forming part of the constitution of said product is selected from those which can be selected by a cell penetration test comprising initial incubation of the immunovector being studied in the presence of cells in the nucleus from which the active principle that can be associated with the immunovector must be transferred followed, after fixing and permeabilization of said cells, by further incubation with labeled anti-immunovector antibodies, and finally by detection in the immediate proximity of the nucleus or in said nucleus of the antigen-antibody type immunological reaction between the immunovector and the anti-immunovector antibody.

4. A product according to any one of claims 1 to 3, **characterized in that** the immunovector is formed from an antibody with an affinity for a nucleic acid or a fragment of said antibody conserving said affinity.

5. A product according to claim 4, **characterized in that** the immunovector is selected from monoclonal IgG, (Fab')2 or (Fab') fragments or any polypeptides corresponding to the antibody site(s) involved in recognizing the corresponding nucleic acid.

6. A product according to claim 4 or claim 5, **characterized in that** the immunovectors are immunoglobulins, in particular IgG carrying anti-DNA activity originating from normal individuals.

7. A product according to claim 4 or claim 5, **characterized in that** the immunovectors are immunoglobulins, in particular IgG carrying anti-DNA activity originating from individuals with auto-immune syndromes, more particularly disseminated lupus erythematosus syndromes.

8. A product according to any one of claims 4 to 7, **characterized in that** the immunovector is a bispecific antibody recognizing DNA and a protein such as Tat, HIV Rev retrovirus, and also surface markers such as CD3, CD4, CD8, CD19 or CD34.

9. A product according to any one of claims 1 to 8, **characterized in that** the active principle coupled to the immunovector is a molecule selected from nucleic acids, proteins and haptenes.

10. A product according to any one of claims 1 to 8, **characterized in that** the active principle coupled to the immunovector is constituted by a plasmid allowing expression of a protein coded by a gene contained in said plasmid.

11. A product according to claim 10, **characterized in that** when the immunovector is an antibody or an antibody fragment, it is coupled in advance to an agent that can induce a compacting effect on the DNA, said agent preferably being polylysine.

12. A product according to any one of claims 1 to 8, **characterized in that** the active principle is constituted by a gene intended to be integrated into the genome of target cells, in particular by homologous recombination, more particularly a naked DNA containing a nucleic acid sequence coding for a polypeptide originally from bacterial or eukaryotic cells, fungus cells, or virus cells, said polypeptide having vaccinating properties.

13. A product according to any one of claims 1 to 8, **characterized in that** said active principle enables immortalization of selected cell types, particularly macrophages, dendritic cells, B and T cells, and haemopoietic cells, in particular of human origin.

14. A product according to any one of claims 1 to 8, **characterized in that** the active principle is an antisense oligonucleotide that can inhibit proteic or nucleotidic synthesis, for example in cells affected by an HIV retrovirus or in tumour cells.

15. Eukaryotic cells containing active molecules, preferably active on the nucleus, **characterized in that** said molecules are not naturally incorporated into the nuclei of said cells, or have a low degree of expression in said cells, said molecules being present in said cells in the immediate proximity of their nuclei or in said nuclei, and coupled to an immunovector **characterized by** its affinity for DNA of said cells, as a product according to any one of claims 1 to 12.

16. Cells according to claim 15, **characterized in that** said cells can be infected by a virus, or tumour cells.

17. Hybridomas deposited at the CNCM on 30^{th} June 1995 with accession number:
I-1605
I-1606
I-1607.

18. A process for in vitro transfer of an active principle into the nuclei of selected eukaryotic cells, **characterized by** coupling said active principle with an immunovector having both the capacity to allow internalization of said active principle into said eukaryotic cells, and an affinity for the DNA of said cells, to render said immunovector capable of transporting said biologically active principle to the immediate proximity or into the nuclei of said cells.

19. A process according to claim 18, **characterized in that** the biologically active principle is covalently or non-covalently coupled to the immunovector.

20. A process according to claim 18 or claim 19, **characterized in that** the immunovector forming part of the constitution of said product is selected from those that can be selected by a cell penetration test comprising initial incubation of the immunovector being studied in the presence of cells into the nucleus of which the active principle that can be associated with the immunovector must be transported, followed after fixing and permeabilization of said cells by further incubation with labeled anti-immunovector antibodies, and finally, detecting in the proximity of the nucleus or in said nucleus of the antigen-antibody type immunological reaction between the immunovector and the anti-immunovector antibody.

21. A process according to claim 18, **characterized in that** the immunovector is formed from an antibody having an affinity for a nucleic acid or a fragment of said antibody conserving said affinity.

22. A process according to claim 21, **characterized in that** the immunovector is selected from antibodies, preferably monoclonal IgG, (Fab')2 or (Fab') fragments, or any polypeptides corresponding to the antibody site(s) involved in recognizing the corresponding nucleic acid.

23. A pharmaceutical composition, **characterized in that** it contains, in association with a physiologically acceptable vehicle, a product according to any one of claims 1 to 9, in which the biologically active principle is an active principle of a drug or vaccine and the immunovector is compatible with the organism of the host for which the drug is intended.

24. Use of a product according to claim 1 for in vitro expression in receiving cells of a nucleotide sequence that is heterologous with respect to the host DNA.
